## Europäisches Patentamt

## European Patent Office

## Office européen des brevets

(19)

(11) Veröffentlichungsnummer: **0 121 816**
**B1**

(12)

# EUROPÄISCHE PATENTSCHRIFT

(45) Veröffentlichungstag der Patentschrift:
**01.06.88**

(51) Int. Cl.⁴: **G 01 N 33/34**

(21) Anmeldenummer: **84102793.1**

(22) Anmeldetag: **14.03.84**

(54) **Verfahren und Vorrichtung zur Prüfung der Durchschreibeeigenschaften von druckempfindlichen Durchschreibepapieren und daraus hergestellten Durchschreibesätzen.**

(30) Priorität: **09.04.83 DE 3312749**

(43) Veröffentlichungstag der Anmeldung:
**17.10.84 Patentblatt 84/42**

(45) Bekanntmachung des Hinweises auf die Patenterteilung:
**01.06.88 Patentblatt 88/22**

(84) Benannte Vertragsstaaten:
**AT BE CH DE FR GB IT LI NL SE**

(56) Entgegenhaltungen:
**DE - A - 2 803 195**
**DE - A - 2 912 056**
**FR - A - 2 344 406**

**SOVIET INVENTIONS ILLUSTRATED, Woche C11,
veröffentlicht 23.04.1980, Zusammenfassung Nr.
19913C11, Derwent, London, GB;
Graphic Product Bulletin (Franz. Büttner Ltd. CH-8132
Egg/2H) "Graphic - Kuli"
Prüfbau - Mehrzweck - Probedruckmaschine (System
Dr. Dürner) 8123 Peissenberg/München**

(73) Patentinhaber: **Feldmühle Aktiengesellschaft,
Fritz-Vomfelde-Platz 4, D-4000 Düsseldorf 11 (DE)**

(72) Erfinder: **Horand, Dieter, Dr. Dipl.-Phys., Heidweg 40,
D-4060 Viersen 12 (DE)**

## Beschreibung

Die vorliegende Erfindung betrifft ein Verfahren und eine Vorrichtung zur Prüfung der Durchschreibeeigenschaften, der Intensität, Strichschärfe, Lesbarkeit und Verschmutzungsneigung von druckempfindlichen Durchschreibepapieren und daraus hergestellten Durchschreibesätzen, bei dem mittels eines mechanisch oder elektromechanisch betätigten Markierungsstiftes mit wählbarer Auflagekraft auf das Durchschreibepapier bzw. den daraus hergestellten Durchschreibesatz ein Strichraster aufgezeichnet wird. Eine derartige Vorrichtung ist aus der DE-A 2 912 056 sowie aus der Firmenschrift der Fr. Büttner AG, CH 8132 Egg, über das Graphic-Kuli-Gerät bekannt.

Einige Qualitätskriterien für druckempfindliche Durchschreibepapiere sind z.B. Schriftintensität, Strichschärfe, Lesbarkeit, Verschmutzungsneigung und Durchschreibeverhalten im Mehrfachsatz, wobei zwischen verschiedenen Anwendungsbereichen unterschieden werden muss, die sich durch charakteristische Durchschreibeenergiebereiche auszeichnen: Handdurchschriften, Schnelldruckdurchschriften, Schreibmaschinendurchschriften.

In allen Fällen handelt es sich um die Übertragung von Schriftzeichen, d.h. von aus schmalen Linien zusammengesetzten Buchstaben, Ziffern usw. und nicht von grösserflächigen Zeichen.

Für die Beurteilung der Schriftintensität allein würde es genügen, vollflächige Durchschriften zu erzeugen, z.B. in Form von Kalanderabdruck. Ebenso sind vollflächige Durchschriften z.B. mit handelsüblichen Probeandruckgeräten erzeugt worden. Diese vollflächigen Abdrucke haben für die Beurteilung der Schriftintensität den Vorteil, dass die Intensität dieses Abdruckes, beispielsweise eine schwarze Durchschrift, durch übliche Messgeräte, wie Remissionsfotometer grossflächig reproduzierbar gemessen werden kann, so dass die fotometrische Auswertung einen über eine grössere Fläche integrierten Wert liefert, der dem Eindruck des menschlichen Auges entspricht.

Der Nachteil so erzeugter vollflächiger Durchschriften, die im allgemeinen zwischen starren Platten oder Walzen erzeugt werden, ist, dass die erhaltenen Durchschriften ungleichmässig sind, da z.B. ungleichmässige Papierformationen, Dikken und/oder Dichteschwankungen zu örtlich schwankenden Druckeinwirkungen führen. Beispielsweise werden beim Kalanderabdruck dünnere und/oder weniger dichte Stellen zwischen benachbarten dickeren und/oder dichteren wenig oder gar nicht belastet, so dass in der Durchschrift eine hellere Stelle resultiert. Das entspricht aber keineswegs den wirklichen Verhältnissen bei der Durchschrift von einzelnen Zeichen, die aufgrund ihrer geringflächigen Abmessung auch solche Bereiche erfassen können.

Will man ausser über die Intensität auch Aussagen über weitere Qualitätskriterien, wie z.B. Strichschärfe und Lesbarkeit erhalten, so müssen einzelne Zeichen geschrieben oder gedruckt werden. Zur Erzeugung der Zeichen werden oft handelsübliche Schnelldrucker oder Schreibmaschinen eingesetzt, wie auch in den ASTM F 591-78 und ASTM F 497-77 beschrieben. ASTM = American National Standard. Im allgemeinen sind diese aber für Testzwecke wegen der schlecht reproduzierbaren Druckkräfte ungeeignet. Diesen Nachteil kann man durch aufwendig konstruierte Laborgeräte umgehen, die im wesentlichen den mechanischen Druckvorgang simulieren.

Mit dem Fogra-Drucktester hat man schon versucht, Merkmale des vollflächigen Prüfverfahrens mit einem solchen, bei dem Einzelzeichen verwendet werden, zu kombinieren. Auch bei diesem sind aber die vorher beschriebenen Nachteile des vollflächigen Verfahrens nicht ausgeräumt, weil sich auch hierbei die Ungleichmässigkeit der Papierformation störend auswirkt und damit zu schlecht reproduzierbaren Resultaten führt.

Ein weiteres Prinzip wurde bei dem sogenannten Graphic-Kuli-Gerät verwirklicht. Hier werden Durchschriften mit Hilfe einer sich kreisförmig bewegenden, belasteten Kugelschreibermine erzeugt, wobei zumindest die Reproduzierbarkeit der Krafteinwirkung gut verwirklicht ist.

Einer der grössten Nachteile von Einzeldurchschriften, die aus einzelnen Schriftzeichen besteht, ist, dass gerade eines der wichtigsten Qualitätskriterien, die Schriftintensität, sehr schwer zu messen ist. Das Problem liegt hier darin, dass bereits in schwacher Vergrösserung ein linienförmiges Schriftbild diskontinuierlich erscheint, da benachbarte Bereiche mit und ohne Farbreaktion erkennbar werden. Wegen des statistischen Charakters dieser Unregelmässigkeiten resultieren starke Schwankungen des fotometrischen Signals mit der Konsequenz, dass für eine sichere Aussage über eine grosse Zahl von Einzelmessungen gemittelt werden muss. Im übrigen entspricht das mikrofotometrische Bild in keiner Weise dem, was das menschliche Auge über einen grösseren Bereich integrierend sieht.

Die bisher bekannten Verfahren zur Prüfung der Durchschreibeeigenschaften von druckempfindlichen Durchschreibepapieren erzeugen entweder relativ grosse Vollflächendurchschriften oder linienförmige Durchschriften mit den bekannten Nachteilen: bei den Vollflächendurchschriften kommt es infolge der durch die Faserverteilung bedingten Anisotropie des Trägerpapiers zu Hell/Dunkel-Erscheinungen, die bei der anschliessenden fotoelektrischen Auswertung stören bzw. den Messwert verfälschen. Bei den Liniendurchschriften ist die Auswertung entweder wie weiter oben beschrieben fotoelektrisch im schwach vergrössernden mikroskopischen Bereich vorzunehmen und damit apparate- und zeitaufwendig, oder man erfasst wie z.B. beim Graphic-Kuli mit der Messöffnung des Fotometers sowohl beschriebene als auch unbeschriebene Flächen. Da dabei der Anteil der weissen Flächen gegenüber den beschriebenen Flächen

2

gross ist, werden nicht die wahren Schriftintensitäten gemessen, sondern ein nicht aussagekräftiger Zwischenwert aus beschrifteter und unbeschrifteter Fläche. Ein objektiver Vergleich der Durchschreibeeigenschaften von druckempfindlichen Durchschreibepapieren ist so nur eingeschränkt oder gar nicht möglich, so dass man sich auf eine visuelle Beurteilung beschränken muss, die mit allen Nachteilen der subjektiven Fehlermöglichkeit erkauft wird.

Aufgabe der vorliegenden Erfindung ist es nun, ein Verfahren und eine Vorrichtung zur Prüfung der Durchschreibeeigenschaften von druckempfindlichen Durchschreibepapieren zu schaffen, das die Vorteile eines vollflächigen Prüfverfahrens mit denen eines strich- oder punktförmigen Prüfverfahrens kombiniert, ohne mit deren Nachteilen behaftet zu sein. Insbesondere sollen die Bedingungen der Erzeugung der Durchschrift weitgehend den Verhältnissen beim Durchschreibevorgang in der Praxis entsprechen. Ausserdem ist es eine weitere Aufgabe dieser Erfindung, neben einer optimalen und objektiven Beurteilung der Intensität auch die Möglichkeit einer gleich guten Beurteilung der Lesbarkeit, Strichschärfe und Verschmutzungsneigung zu ermöglichen, wobei die Auswertung mit handelsüblichen, in der Praxis bewährten Geräten, wie beispielsweise Remissionsfotometern, ohne grossen Zeitaufwand durchgeführt werden kann.

Zur Lösung dieser Aufgabe sieht die Erfindung ein Verfahren zur Prüfung der Durchschreibeeigenschaften, der Intensität, Strichschärfe, Lesbarkeit und Verschmutzungsneigung von druckempfindlichen Durchschreibepapieren und daraus hergestellten Durchschreibesätzen, bei dem mittels eines mechanisch oder elektromechanisch betätigten Markierungsstiftes mit wählbarer Auflagekraft auf das Durchschreibepapier bzw. den daraus hergestellten Durchschreibesatz ein Strichraster aufgezeichnet wird, das dadurch gekennzeichnet ist, dass auf das Durchschreibepapier einzelne Striche nahe beieinanderliegend, sich jedoch nicht kreuzend, aufgebracht werden und dass das erzeugte Raster mittels fotoelektrischer Remissionsmessung ausgewertet wird, vor. Die Erfindung sieht auch eine Vorrichtung gemäss dem Anspruch 5 bzw. 6 vor.

Mit dem erfindungsgemässen Verfahren werden die Nachteile der bisher bekannten Prüfverfahren, nämlich ungleichmässige Vollflächendurchschriften, erforderliche Auswertung von Einzeldurchschriften im mikroskopischen Bereich, schlecht reproduzierbare Messergebnisse und nur subjektiv beurteilbare Qualitätskriterien, wie z.B. die Lesbarkeit einer Durchschrift, überwunden. Es wurde ein Prüfverfahren geschaffen, das neben seinem Messkomfort beliebig oft reproduzierbare objektive Kriterien zur Beurteilung der Qualitätseigenschaften von Durchschreibepapieren liefert.

Die gemäss der Erfindung auf das Durchschreibepapier aufgebrachten Striche, die nahe beieinander liegen, sich jedoch nicht kreuzen, stellen Einzelflächen dar, die eine endliche, von der Spitze des Markierungsstiftes mitbestimmte Breitenausdehnung besitzen. Der Abstand der einzelnen Striche zueinander ist dabei definiert als der Abstand der einzelnen Strichmitten untereinander. Wird der Abstand nun bevorzugt so gewählt, dass ein Strichraster entsteht, dessen Einzelstriche vom menschlichen Auge nicht mehr aufgelöst werden kann, so entsteht eine Vollfläche. Dabei werden zweckmässig die aus Einzelstrichen bestehenden, dem menschlichen Auge aber als Vollfläche erscheinenden Durchschriften durch eine oszillierende Bewegung eines belasteten Markierungsstiftes und gleichzeitigen Vorschub des zu prüfenden Durchschreibepapieres bzw. des daraus hergestellten Durchschreibesatzes oder des Markierungsstiftes erzeugt, wobei Papier/Markierungsstiftvorschub und Frequenz des Markierungsstiftes aufeinander abgestimmt sind. Man erhält so beliebig grosse Vollflächendurchschriften, die sich durch ausserordentliche Gleichmässigkeit auszeichnen. Die Anisotropie der Trägerpapiere stört nicht. Es treten keine fleckigen Vollflächendurchschriften auf, wie sie z.B. beim Abdruck zwischen starren Walzen oder Platten erhalten wurden. Zweckmässigerweise wird dazu der Abstand der einzelnen Strichmitten so gewählt, dass er 30 bis 150% der Strichbreiten beträgt. Die einzelnen Striche, die das Strichraster bilden, verlaufen zueinander parallel oder in einem spitzen Winkel. Beide Arten der Anordnung haben sich bewährt. Verlaufen die Striche in einem spitzen Winkel zueinander, stellt die Schreibspur des Markierungsstiftes also eine Zickzacklinie dar, ist der Abstand der Strichmitten in der Mitte zwischen zwei Umkehrpunkten der Zickzacklinie zwischen 30 und 150% der Strichbreite festzulegen.

Eine für die elektrische Remissionsmessung ausreichende Vollfläche wird dadurch erhalten, dass der Abstand der einzelnen Strichmitten zueinander 30 bis 150% der Strichbreite beträgt. Die Länge und Breite der so erzeugten Vollfläche bleibt zunächst freigestellt, sie ist nur von dem Durchmesser der Messöffnung des Remissionsfotometers abhängig. Zweckmässigerweise wird man die Breite der zu erzeugenden Vollfläche so wählen, dass sie etwas grösser ist als die Messöffnung. Die Länge der Vollfläche wird zweckmässigerweise so gewählt, dass sie ein vielfaches des Durchmessers der Messöffnung ist. Somit erhält man für die Messung mehrere nebeneinander liegende Messflächen. Beträgt z.B. der Durchmesser der Messöffnung eines Fotometers 30 mm, so wählt man die Länge der zu erzeugenden Vollfläche zu 90 mm. Damit erhält man dann 3 separate Messflächen.

Um die verschiedenen Druckkräfte der in der Praxis vorkommenden Schreibdrucke, wie Handdurchschrift, Schnelldrucker und Schreibmaschine bei der Erzeugung der Vollflächendurchschriften reproduzierbar nachzustellen, wird der Markierungsstift mit einer senkrechten Auflagekraft von 0,1 bis 5,0 N über das zu beschriftende Durchschreibepapier bzw. den daraus hergestellten Durchschreibesatz geführt. Zur Auswertung der Intensität werden die durchgeschriebenen Vollflächen auf den Kopien des Durchschreibesatzes herangezogen. Besteht der Durchschreibesatz aus sogenanntem Einschichtpapier (selfcontained-paper), kann auch die Vollfläche auf dem Deckblatt des Durchschreibesatzes zur Messung verwendet werden.

Die Schriftintensität so erzeugter Vollflächendurchschriften kann sehr einfach gemessen werden, indem man die Helligkeitsdifferenz $\Delta y$ zwischen beschrifteter und unbeschrifteter Fläche bestimmt, oder aber man gibt die Intensität als Quotient aus der Differenz der Helligkeit $\Delta y$ zu der Helligkeit y der unbeschrifteten Fläche in % an. Mit dem beschriebenen Verfahren erhält man für eine bestimmte Durchschreibepapiersorte einen reproduzierbaren Intensitätswert. Variiert man nun noch die Auflagekraft des Markierungsstiftes innerhalb des Bereiches von 0,1 bis 5,0 N bei der Erzeugung der Durchschriften, so erhält man im Fall eines Mehrfachsatzes bei Auftragen der fotometrisch ausgemessenen Schiftintensitäten gegen die Auflagekraft für jeden Durchschlag eine Kurve. Diese Kurvenscharen sind praktisch als Fingerabdruck für ein Durchschreibepapier mit bestimmten Qualitätseigenschaften anzusehen. Werden die Kurvenscharen zu geringen Auflagekräften extrapoliert, so wird gleichzeitig die Verschmutzungsanfälligkeit des Durchschreibepapieres charakterisiert. An einer Bestimmung der Verschmutzungsanfälligkeit besteht deshalb ein technisches Interesse, weil durch unbeabsichtigte, geringe Druckanwendungen und Stösse beim Arbeiten mit diesen Papieren solche unerwünschten Verschmutzungen entstehen können, die um so grösser sind, je empfindlicher solche Papiere zur Verfärbung schon bei niedrigen Drucken neigen.

Die Lesbarkeit einer mit einem Durchschreibepapier erzeugten Durchschrift hängt von der Intensität und der Strichschärfe der erzeugten und durchgeschriebenen Zeichen ab. Eine hohe Intensität in Verbindung mit guter Strichschärfe bedeutet gute Lesbarkeit, eine niedrige Intensität und schlechte Strichschärfe bedeutet schlechte Lesbarkeit. Die mit einem bestimmten Durchschreibepapier erzielbare Strichschärfe wird hauptsächlich durch die Rohpapierqualität und die Eigenschaften der darauf aufgebrachten Beschichtungen bei Druckeinwirkung durch das Beschreibmittel bestimmt. Will man die mit einem Durchschreibepapier erzielbare Strichschärfe messen, so wird man die Einzelstriche so anordnen, dass der Abstand der einzelnen Strichmitten 100 bis 150% der Strichbreite beträgt. Aufgrund des Druckkegels, der in dem zu prüfenden Durchschreibesatz durch den schreibenden Markierungsstift aufgebaut wird, werden die auf dem Deckblatt des Durchschreibesatzes noch als Einzelstriche wahrnehmbaren Striche in den nachfolgenden Blättern des Satzes sich zunehmend verbreitern und schliesslich überlappen, so dass Rückschlüsse auf die Beeinflussung der Strichschärfe durch das zu prüfende Papier gezogen werden können. Wird zur Beurteilung der Strichschärfe anstelle eines Rasters mit parallel zueinander verlaufenden Strichen ein Raster mit spitzwinklig zueinander verlaufenden Strichen gewählt, deren Abstand voneinander ebenfalls 100 bis 150% der Strichbreite beträgt, erfolgt die Auswertung so, dass die Flächen in den Zwickeln, die durch die spitzwinklig zueinander verlaufenden Striche gebildet werden, beurteilt werden.

Für das erfindungsgemässe Verfahren geeignete Vorrichtungen werden nachfolgend anhand der Figurenbeschreibung erläutert. Eine vorteilhafte Vorrichtung zur Durchführung des Verfahrens besteht aus einer mechanisch betätigten Vorrichtung mit in X-Richtung beweglicher Auflageplatte für das zu prüfende Durchschreibepapier bzw. den daraus hergestellten Durchschreibesatz und einem in Y-Richtung beweglich geführten Markierungsstift, der zweckmässig mit austauschbaren Zusatzgewichten belastet werden kann, um Auflagekräfte von beispielsweise 0,1 N bis zu 5,1 N in Stufen zu 0,5 N zu erreichen. Als Markierungsstift ist jede Art von Stift einsetzbar, jedoch hat es sich als vorteilhaft erwiesen, nur solche Stifte zu verwenden, deren Spitze eine punktförmige Berührung mit der Papieroberfläche gewährleistet, wobei die Spitze keinem oder nur einem minimalen Verschleiss unterliegt und wobei die Spitze mit einer rollenden Reibung über das zu prüfende Papier gleitet. Dafür haben sich insbesondere Kugelschreiberminen nach DIN, wie z.B. Pelikan Perfekt 237 M, bewährt. Der Markierungsstift ist in einer Halterung vertikal beweglich geführt, so dass er in dieser unter dem Einfluss eines der Zusatzgewichte, die auf das der Schreibspitze abgewandte Ende aufgesetzt werden können, reibungsfrei gleiten kann. Die Halterung ist an einer Geradführung angelenkt und kann an dieser in Y-Richtung über die Auflageplatte mit dem darauf befindlichen Durchschreibesatz hinwegbewegt werden. Zur oszillierenden Bewegung der mit dem Markierungsstift verbundenen Halterung entlang der Geradführung wird ein erstes Koppelgetriebe benutzt. Als Koppelgetriebe sind grundsätzlich alle bekannten mechanischen Getriebevorrichtungen einsetzbar, die imstande sind, eine konstante Drehbewegung in eine geradlinige Bewegung umzusetzen. Beispielsweise seien genannt: Kurbelgetriebe, Exzentergetriebe, Steuerwalzengetriebe, Zugband- oder Zugkettengetriebe.

Die Hublänge wie auch die Geschwindigkeit der geradlinigen Bewegung des Markierungsstiftes wird durch das erste Koppelgetriebe erzeugt. Sollen Hublänge und/oder Geschwindigkeit der geradlinigen Bewegung des Markierungsstiftes in Y-Richtung verändert werden, erfolgt dieses mittels einer Änderung des Kurbelhalbmessers oder der Steuerwalzengeometrie oder der Drehzahl des Zugband- oder Kettengetriebes sowie eine Verlängerung oder Verkürzung des Zugbandes. Für den Vorschub der Auflageplatte in X-Richtung ist ein zweites Koppelgetriebe mit dieser kraftschlüssig verbunden. Dazu wird die Auflageplatte mit Vorrichtungen wie Zahnstangen, Durchbrechungen u.ä. versehen. Das zweite Koppelgetriebe ist mit einer Einrichtung zur Veränderung der Abtriebsdrehzahl ausgerüstet. Diese besteht vorzugsweise aus einer veränderlichen Räderübersetzung, um die lineare Vorschubgeschwindigkeit der Auflageplatte in X-Richtung in den gewünschten Bereichen verstellen zu können. Wird ein schrittweiser Vorschub der Auflageplatte in X-Richtung gewünscht, ist das zweite Koppelgetriebe vorzugsweise als Schrittschaltwerk durchzubilden, so z.B. als Sperrklinkenantrieb oder Malteserkreuzantrieb. Es hat sich als besonders zweckmässig erwiesen, erstes und zweites Koppelgetriebe kraftschlüssig miteinander zu verbinden, da damit gewährleistet ist, dass die von beiden Getrieben erzeugten Bewegungen immer in einem konstanten Verhältnis zueinander stehen und somit reproduzierbare Strichraster erzeugt werden können.

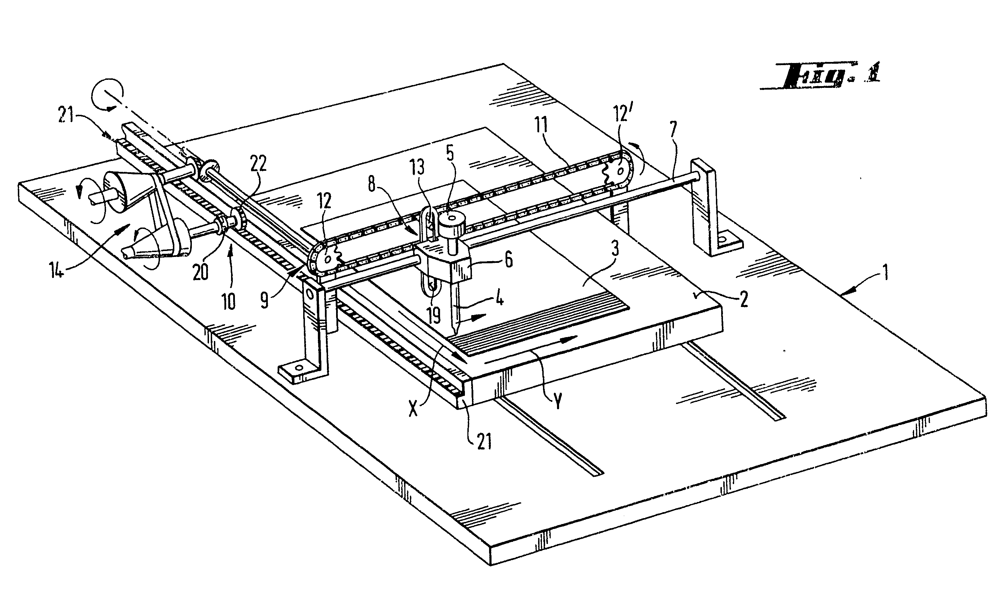

Figur 1 zeigt eine bevorzugte Ausführung der mechanischen Vorrichtung zur Durchführung des Verfahrens, ohne dass die Erfindung auf diese Ausführung beschränkt ist. Auf einer Grundplatte 1 ist die in X-Richtung beweglich geführte Aufnahmeplatte 2 für den zu prüfenden Durchschreibesatz 3 angebracht. Mit dem Durchschreibesatz 3 steht der Markierungsstift 4, der mit Zusatzgewichten 5 belastet ist, in Berührung. Der Markierungsstift 4 selbst ist mittels der Halterung 6 an der Geradführung 7 angelenkt. Die Geradführung 7 ist wiederum mit der Grundplatte 1 mittels zweier senkrechter Stützen verbunden. Die Halterung 6 weist einen Mitnehmer 8 auf, der mit dem Koppelgetriebe 9 in kraftschlüssiger Verbindung steht, das die Bewegung der Halterung 6 mit dem darin geführten Markierungsstift 4 in Y-Richtung bewerkstelligt. Das Koppelgetriebe 9 ist eine endlose Rollenkette 11, die um zwei Kettenräder 12 und 12' herumgeführt ist. Ein an der Rollenkette 11 befestigter Dorn 13 greift in einen im Mitnehmer 8 befindlichen Schlitz 19 ein und bewirkt so die Hin- und Herbewegung des Markierungsstiftes 4. Mit dem Kettenrad 12 steht ein Wechselgetriebe 14 in Verbindung, das den Vorschub der Auflageplatte 3 über das Koppelgetriebe 10, das aus einem Schrittschaltwerk besteht, in der gewünschten Vorschubgeschwindigkeit in X-Richtung so bewerkstelligt, dass bei jeder Bewegungsumkehr des Markierungsstiftes 4 die Auflageplatte 2 um einen mittels dem Getriebe 10 vorgewählten Betrag in X-Richtung verschoben wird.

Eine weitere vorteilhafte Vorrichtung zur Durchführung des Verfahrens besteht aus einer elektromechanisch oder elektrisch betätigten Vorrichtung, die aus einem handelsüblichen Y-t-Registrierschreiber und einem elektrischen Sender zusammengesetzt ist, wobei dieser aus einem handelsüblichen Frequenzgenerator besteht, der die erforderlichen elektrischen Signale für die Betätigung des in Y-Richtung beweglichen Markierungsstiftes erzeugt. Der Sender ist mit den Eingangsklemmen des Registrierschreibers elektrisch leitend verbunden. Anstelle der Schreibfeder des Registrierschreibers wird der Markierungsstift an die Bewegungsvorrichtung mittels einer Halterung beweglich angelenkt, so dass der Markierungsstift sowohl der Y-Komponente folgen kann als auch in vertikaler Z-Richtung frei beweglich ist. Dabei hat es sich als zweckmässig erwiesen, die ursprünglich vorhandene Schreibfederhalterung durch eine mechanisch verstärkte Halterung zu ersetzen, die im Stande ist, eine sichere Führung des Markierungsstiftes zu gewährleisten und die erforderlichen Beschleunigungskräfte auf den mit Gewichten belasteten Markierungsstift zu übertragen. Im Falle eines Y-t-Registrierschreibers ist dieser Vorschub bei handelsüblichen Geräten bauseitig bereits in Form des Registrierpapiervorschubes integriert. Dabei hat es sich als nützlich erwiesen, mehrere Vorschubgeschwindigkeiten zur Verfügung zu haben. Ganz besonders vorteilhaft hat sich ein mit einem elektrischen Sender verbundener X-Y-Registrierschreiber zur Durchführung des Verfahrens bewährt. Bei dieser Vorrichtung wird der Markierungsstift über das Durchschreibepapier bzw. den daraus hergestellten Durchschreibesatz in X- und Y-Richtung hinwegbewegt, während das zu prüfende Durchschreibepapier auf der ortsfesten Auflageplatte des Registrierschreibers unverrückbar befestigt ist. Die Bewegung des Markierungsstiftes wird wiederum von dem elektrischen Sender mittels elektrischer Signale veranlasst, wobei der Sender zwei Signalausgänge besitzt, deren einer die Y-Komponente, der andere die X-Komponente des Registrierschreibers ansteuert. Die zur Betätigung des Markierungsstiftes in Y-Richtung erforderlichen elektrischen Signale werden von dem elektrischen Sender in Form von Dreiecks-, Rechtecks- oder Sinussignalen als variable Spannungen U oder variable Stromstärken I erzeugt. Alle drei Signalformen haben sich grundsätzlich bewährt, jedoch wählt man vorteilhafterweise das Dreieckssignal, da bei dieser Signalform der elektromechanische Antrieb für die Bewegung des Markierungsstiftes aufgrund der aufzubringenden Beschleunigungskräfte nur gering mechanisch belastet wird. Zur Bewegung des Markierungsstiftes in X-Richtung wird von dem elektrischen Sender ein linear oder stufenförmig ansteigendes Signal in Form einer Spannung U oder einer Stromstärke I erzeugt. Ein lineares Signal bewirkt eine konstante Bewegung, ein stufenförmiges Signal eine schrittweise Bewegung des Markierungsstiftes in X-Richtung.

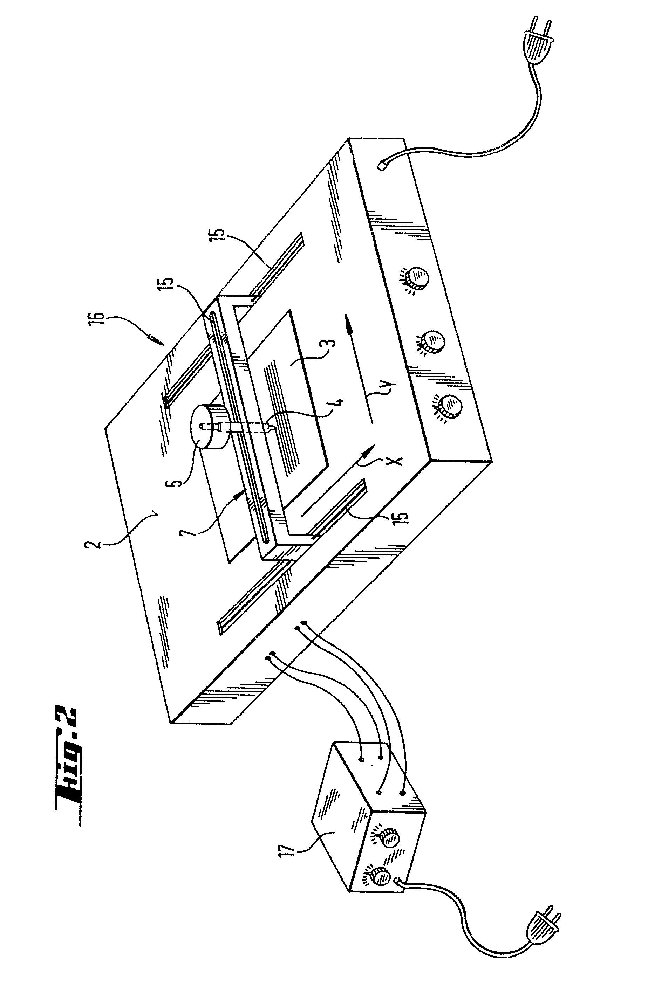

Figur 2 zeigt eine elektrische Vorrichtung zur Durchführung des Verfahrens, ohne dass die Erfindung auf diese Ausführung beschränkt ist. Ein elektrischer Sender 17, der von einer nicht gezeigten Stromquelle gespeist wird, erzeugt Signale für die Bewegungen in X- und Y-Richtung und speist diese Signale mittels elektrischer Kabelverbindungen in den elektrischen Registerschreiber 16 ein. Auf der Auflageplatte 2 des elektrischen Registerschreibers 16 ist der zu prüfende Durchschreibesatz 3 unverrückbar befestigt. Über diesen Durchschreibesatz 3 bewegt sich die Geradführung 7 in X-Richtung mit dem in Y-Richtung oszillierenden Markierungsstift 4, der durch Zusatzgewichte 5 belastet ist, hinweg und erzeugt dabei das gewünschte Strichraster.

**Patentansprüche**

1. Verfahren zur Prüfung der Durchschreibeeigenschaften, der Intensität, Strichschärfe, Lesbarkeit und Verschmutzungsneigung von druckempfindlichen Durchschreibepapieren und daraus hergestellten Durchschreibesätzen, bei dem mittels eines mechanisch oder elektromechanisch betätigten Markierungsstiftes (4) mit wählbarer Auflagekraft auf das Durchschreibepapier bzw. dem daraus hergestellten Durchschreibesatz (3) ein Strichraster aufgezeichnet wird und dass das erzeugte Raster mittels fotoelektrischer Remissionsmessung ausgewertet wird, dadurch gekennzeichnet, dass auf das Durchschreibepapier einzelne Striche nahe beieinanderliegend, sich jedoch nicht kreuzend, aufgebracht werden.

2. Verfahren nach Anspruch 1, dadurch gekennzeichnet, dass die einzelnen Striche so aufgebracht werden, dass der Abstand der einzelnen Strichmitten zueinander 30 bis 150% der Strichbreite beträgt.

3. Verfahren nach Anspruch 1 und 2, dadurch gekennzeichnet, dass die einzelnen Striche parallel zueinander verlaufend aufgebracht werden.

4. Verfahren nach Anspruch 1 bis 3, dadurch gekennzeichnet, dass die einzelnen Striche zueinander in einem spitzen Winkel verlaufend aufgebracht werden.

5. Vorrichtung zur Prüfung der Durchschreibeeigenschaften, der Intensität, Strichschärfe, Lesbarkeit und Verschmutzungsneigung von druckempfindlichen Durchschreibepapieren und daraus hergestellten Durchschreibesätzen, bei dem mittels eines mechanisch oder elektromechanisch betätigten Markierungsstiftes (4) mit wählbarer Auflagekraft auf das Durchschreibepapier bzw. dem daraus hergestellten Durchschreibesatz (3) ein Strichraster aufgezeichnet, gekennzeichnet durch eine in X-Richtung bewegliche Auflageplatte (2) für den zu prüfenden Durchschreibesatz (3), einen in Y-Richtung beweglich geführten mit unterschiedlichen Auflagekräften beaufschlagbaren Markierungsstift (4) und Mitteln zur koordinierten Bewegung der Auflageplatte (2) und des Markierungsstiftes (4) zur Erzeugung von einzelnen, nahe beieinanderliegenden, sich jedoch nicht kreuzenden Strichen.

6. Vorrichtung zur Prüfung der Durchschreibeeigenschaften, der Intensität, Strichschärfe, Lesbarkeit und Verschmutzungsneigung von druckempfindlichen Durchschreibepapieren und daraus hergestellten Durchschreibesätzen, bei dem mittels eines mechanisch oder elektromechanisch betätigten Markierungsstiftes (4) mit wählbarer Auflagekraft auf das Durchschreibepapier bzw. dem daraus hergestellten Durchschreibesatz (3) ein Strichraster aufgezeichnet, gekennzeichnet durch eine fest angeordnete Auflageplatte (2) für den zu prüfenden Durchschreibesatz (3), einen sowohl in Y-Richtung als auch X-Richtung beweglich geführten mit unterschiedlichen Auflagekräften beaufschlagbaren Markierungsstift (4) und Mitteln zur koordinierten Bewegung des Markierungsstiftes (4) zur Erzeugung von einzelnen, nahe beieinanderliegenden, sich jedoch nicht kreuzenden Strichen.

7. Vorrichtung nach einem der Ansprüche 5 und 6, dadurch gekennzeichnet, dass der Markierungsstift (4) an einer Geradführung (7) mittels einer Halterung (6) beweglich angelenkt ist.

8. Vorrichtung nach Anspruch 5 bis 7, gekennzeichnet durch ein erstes Koppelgetriebe (9) zur oszillierenden Bewegung des Markierungsstiftes (4) in Y-Richtung und ein zweites Koppelgetriebe (10) zum Vorschub der Auflageplatte (2) oder des Markierungsstiftes (4) in X-Richtung.

9. Vorrichtung nach einem der Ansprüche 5 bis 8, dadurch gekennzeichnet, dass die Hublänge und/oder die Abtriebsdrehzahl des Koppelgetriebes (9) einstellbar ist.

10. Vorrichtung nach einem der Ansprüche 8 und 9, dadurch gekennzeichnet, dass die Abtriebsdrehzahl des Koppelgetriebes (10) einstellbar ist.

11. Vorrichtung nach einem der Ansprüche 9 und 10, dadurch gekennzeichnet, dass das zweite Koppelgetriebe (10) als Schrittschaltwerk ausgebildet ist.

12. Vorrichtung nach einem der Ansprüche 5 und 8 bis 11, dadurch gekennzeichnet, dass das erste Koppelgetriebe (9) mit dem zweiten Koppelgetriebe (10) mittels eines mechanischen Wechselgetriebes (14) in kraftschlüssiger Verbindung steht.

13. Vorrichtung nach einem der Ansprüche 5 bis 12, dadurch gekennzeichnet, dass ein elektrische Signale erzeugender Sender mit Antriebsaggregaten für die Bewegung in Y- und/oder X-Richtung gekoppelt ist.

**Claims**

1. Method for testing the print-through quality, the intensity, line sharpness, legibility and tendency to smudging of pressure-sensitive self-copying papers and sets of copies made therefrom, in which a pattern of lines is drawn on the self-copying paper or the set (3) of copies made therefrom by means of a mechanical or electromechanical marking pen (4) with selectable stylus force, characterised in that individual lines, close together, but not crossing, are made on the self-copying paper and that the pattern produced is evaluated by means of photoelectric reflectance measurement.

2. Method according to claim 1, characterised in that the individual lines are made in such a manner that the distance between the centres of the individual lines is from 30 to 150% of the width of the lines.

3. Method according to claims 1 and 2, characterised in that the individual lines are made parallel to one another.

4. Method according to claims 1 to 3, characterised in that the individual lines are made to run together to form a sharp angle.

5. Apparatus for testing the print-through quality, the intensity, line sharpness, legibility and tendency to smudging of pressure-sensitive self-copying papers and sets of copies made therefrom, in which a pattern of lines is drawn on the self-copying paper or on the set (3) of copies made therefrom by means of a mechanical or electromechanical marking pen (4) with selectable stylus force, characterised by a support plate (2), movable in the direction X, for the set (3) of copies that is to be tested, a marking pen (4) that can be subjected to different stylus forces and is guided so that it is movable in the direction Y, and means for moving in a coordinated manner the support plate (2) and the marking pen (4) to produce individual lines that are close together but do not cross.

6. Apparatus for testing the print-through quality, the intensity, line sharpness, legibility and tendency to smudging of pressure-sensitive self-copying papers and sets of copies made therefrom, in which a pattern of lines is drawn on the self-copying paper or on the set (3) of copies made therefrom by means of a mechanical or electromechanical marking pen (4) with selectable stylus force, characterised by a fixed support plate (2) for the set (3) of copies to be tested, a marking pen (4) that can be subjected to different stylus forces and is guided so that it is movable both in the direction Y and in the direction X, and means for moving in a coordinated manner the marking pen (4) for producing individual lines that are close together but do not cross.

7. Apparatus according to claim 5 or claim 6, characterised in that the marking pen (4) is articulated to a slide bar (7) by a holding means (6) in such a manner as to be movable.

8. Apparatus according to claims 5 to 7, characterised by a first coupling gear (9) for the oscillating movement of the marking pen (4) in the direction Y and a second coupling gear (10) for advancing the support plate (2) or the marking pen (4) in the direction X.

9. Apparatus according to any one of claims 5 to 8, characterised in that the stroke length and/or the output speed of the coupling gear (9) is adjustable.

10. Apparatus according to claim 8 or claim 9, characterised in that the output speed of the coupling gear (10) is adjustable.

11. Apparatus according to claim 9 or claim 10, characterised in that the second coupling gear (10) is designed as a step-by-step mechanism.

12. Apparatus according to any one of claims 5 and 8 to 11, characterised in that the first coupling gear (9) is connected in a non-positive manner to the second coupling gear (10) by means of a mechanical change gear (14).

13. Apparatus according to any one of claims 5 to 12, characterised in that a transmitter that produces electrical signals is coupled to drive units for the movement in the directions Y and/or X.

## Revendications

1. Procédé d'essai des propriétés autocopiantes, de l'intensité, de la netteté des traits, de la facilité de lecture et de la tendance à l'encrassement de papiers carbone sensibles à la pression et d'ensembles autocopiants constitués par ces papiers, dans lequel, au moyen d'une pointe de marquage (4) actionnée mécaniquement ou électromécaniquement, on trace avec une force d'application facultative un réseau de traits sur le papier carbone ou sur l'ensemble autocopiant (3) constitué de tels papiers, caractérisé par le fait que sur le papier carbone, on applique différents traits disposés à proximité les uns des autres, mais ne se croisant pas, et qu'on interprète le réseau obtenu au moyen d'une mesure photoélectrique du facteur de luminance.

2. Procédé selon la revendication 1, caractérisé par le fait qu'on applique les différents traits de telle sorte que la distance des milieux de différents traits entre eux représente de 30 à 150% de la largeur des traits.

3. Procédé selon les revendications 1 et 2, caractérisé par le fait qu'on applique les différents traits de telles sorte qu'ils soient disposés parallèlement entre eux.

4. Procédé selon les revendications 1 à 3, caractérisé par le fait qu'on applique les différents traits de telle sorte qu'ils soient disposés sous un angle aigu entre eux.

5. Dispositif pour l'essai des propriétés autocopiantes, de l'intensité, de la netteté des traits, de la facilité de lecture et de la tendance à l'encrassement de papiers carbone sensibles à la pression et d'ensembles autocopiants constitués par ces papiers, dans lequel, au moyen d'une pointe de marquage (4) actionnée mécaniquement ou électromécaniquement, on trace avec une force d'application facultative un réseau de traits sur le papier carbone ou sur l'ensemble autocopiant (3) constitué de tels papiers, caractérisé par un plaque d'appui (2) déplaçable dans le sens X pour l'ensemble autocopiant (3) à examiner, une pointe de marquage (4) déplaçable dans le sens Y et soumise à différentes forces d'application et des moyens pour diriger le mouvement coordonné de la plaque d'appui (2) et de la pointe de marquage (4) en vue de la production de différents traits disposés à proximité les uns des autres, mais ne se croisant pas.

6. Dispositif pour l'essai des propriétés autocopiantes, de l'intensité, de la netteté des traits, de la facilité de lecture et de la tendance à l'encrassement de papiers carbone sensibles à la pression et d'ensembles autocopiants constitués par ces papiers, dans lequel, au moyen d'une pointe de marquage (4) actionnée mécaniquement ou électromécaniquement, on trace avec une force d'application facultative, un réseau de traits sur le papier carbone ou sur l'ensemble autocopiant (3) constitué de tels papiers, caractérisé par une plaque d'appui fixe (2) pour l'ensemble autocopiant (3) à examiner, une pointe de marquage (4) déplaçable aussi bien dans le sens Y que dans le sens X et soumise à différentes forces d'application et des moyens pour diriger le déplacement coordonné de la pointe de marquage (4) en vue de la production de différents traits disposés à proximité les uns des autres, mais ne se croisant pas.

7. Dispositif selon l'une des revendications 5 et 6, caractérisé par le fait que la pointe de marquage (4) s'articule sur un guidage droit (7) au moyen d'une fixation (6).

8. Dispositif selon les revendications 5 à 7, caractérisé par un premier mécanisme à bielle 9 pour le mouvement oscillant de la pointe de marquage (4) dans le sens Y et un second mécanisme à bielle (10) pour l'avance de la plaque d'appui (2) ou de la pointe de marquage (4) dans le sens X.

9. Dispositif selon l'une des revendications 5 à 8, caractérisé par le fait que la longueur de la course et/ou la vitesse de sortie du mécanisme à bielle (9) est réglable.

10. Dispositif selon l'une des revendications 8 et 9, caractérisé par le fait que la vitesse de sortie du mécanisme à bielle (10) est réglable.

11. Dispositif selon l'une des revendications 9 et 10, caractérisé par le fait que le second mécanisme à bielle (10) est réalisé sous la forme d'un mécanisme pas à pas.

12. Dispositif selon l'une des revendications 5 et 8 à 11, caractérisé par le fait que le premier mécanisme à bielle (9) est raccordé par une liaison par friction au second mécanisme à bielle (10) au moyen d'une transmission à changement de vitesse (14).

13. Dispositif selon l'une des revendications 5 à 12, caractérisé par le fait qu'un émetteur produisant des signaux électriques est accouplé à l'unité d'entraînement pour le déplacement dans le sens Y et/ou X.

Fig. 1

0 121 816

Fig.2

0 121 816